# EUROPÄISCHE PATENTANMELDUNG

(11) **EP 1 614 674 A2**
(43) Veröffentlichungstag der Anmeldung: **11.01.2006**
(21) Anmeldenummer: 05011539.3
(22) Anmeldetag: 27.05.2005
(51) Int. Cl.: C07C 211/42, C07C 229/46, B01J 31/22, B01J 31/28

(54) **Tropylidenamine und deren Verwendung**

(30) Priorität: 08.06.2004 DE 102004027771
(71) Anmelder: LANXESS Deutschland GmbH, 51369 Leverkusen (DE)
(72) Erfinder: Grützmacher, Hansjörg, Professor Dr., 8907 Wettswil (CH); Büttner, Torsten, Dr., 07607 Eisenberg (DE); Maire, Pascal, Dr., 4410 Liestal (CH); Ramseier, Maaike, 8057 Zürich (CH); Scheschkewitz, David, Dr., 97074 Würzburg (DE); Zweifel, Theo, 6300 Zug (CH)

(57) **Zusammenfassung**

Die vorliegende Erfindung betrifft Tropylidenamine, ein Verfahren zu ihrer Herstellung sowie deren Verwendung in der Katalyse.

## Beschreibung

Die vorliegende Erfindung betrifft Tropylidenamine, ein Verfahren zu ihrer Herstellung sowie deren Verwendung in der Katalyse.

Aus Deblon (Dissertation Nr. 13920, ETH Zürich, 2000, Kapitel 5) und Maire (Dissertation Nr. 14396, ETH Zürich, 2001) ist bekannt, dass Übergangsmetallkomplexe von Olefin-Phosphanverbindungen für homogenkatalytische Reaktionen wie insbesondere Hydrierungen und Hydrosilylierungen besonders geeignet sind.

Für industrielle Anwendungen wäre es jedoch vorteilhaft, auf die oft teuren und oxidationsempfindlichen Phosphane verzichten zu können. Es bestand daher das Bedürfnis, ein Katalysatorsystem und dafür geeignete Liganden bereitzustellen, das ohne den Einsatz von Phosphanen auskommt und gute Performance in katalytischen Reaktionen zeigt.

Es wurden nun Verbindungen der Formel (I) gefunden, 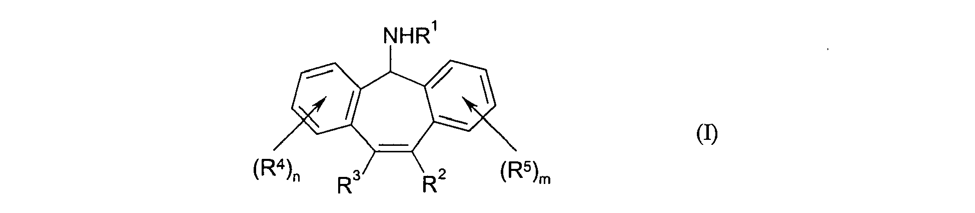 in der
- R¹: entweder für einen Rest der Formel (II) steht 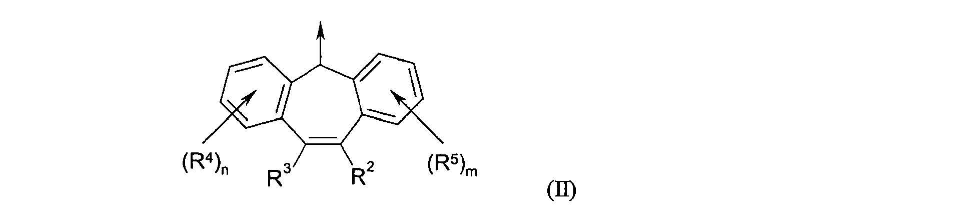 oder für einen Rest der Formel (III), 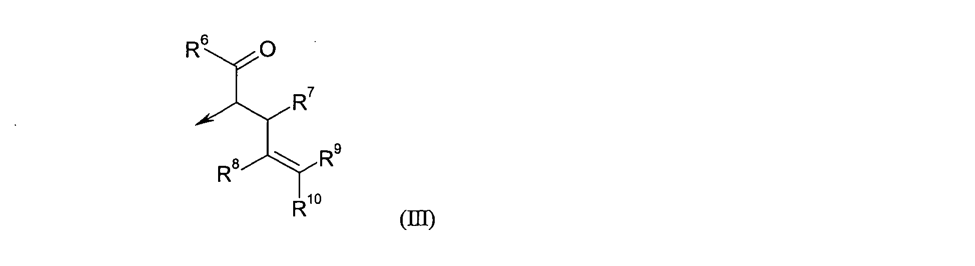
wobei in den Formeln (I), (II) und (III)
die Pfeile jeweils die Bindung des gesamten Restes zum Stickstoffatom anzeigen oder, sofern sie in die Mitte eines aromatischen Systems zeigen, eine Bindung der jeweiligen Reste zum aromatischen Gerüst in beliebiger Position,
R² und R³ jeweils unabhängig voneinander für Wasserstoff, Cyano, Fluor, Chlor, Brom, Iod, C₁-C₁₈-Alkyl, C₄-C₂₄-Aryl, C₅-C₂₅-Arylalkyl, CO₂M, wobei M für ein Alkalimetallion oder ein gegebenenfalls organisches Ammoniumion stehen kann, CONH₂, SO₂N(R¹¹)₂, wobei R¹¹ für Wasserstoff, C₁-C₁₂-Alkyl, C₄-C₁₄-Aryl oder C₅-C₁₅-Arylalkyl steht, SO₃M oder für Reste der Formel (IV) stehen,

T-Het-R¹² (IV)

in der
T fehlt oder für Carbonyl steht,
Het für Sauerstoff oder NR¹¹ steht,
R¹² für C₁-C₁₈-Alkyl, C₄-C₂₄-Aryl oder C₅-C₂₅-Arylalkyl oder N(R¹²)₂ als Ganzes für einen 5 oder 6 gliedrigen cyclischen Aminorest steht und
n und m jeweils unabhängig voneinander für 0, 1, 2 oder 3 stehen und
R⁴ und R⁵ jeweils unabhängig voneinander ausgewählt sind aus der Gruppe Fluor, Chlor, Brom, Iod, Nitro, freies oder geschütztes Formyl, C₁-C₁₂-Alkyl, C₁-C₁₂-Alkoxy, C₁C₁₂-Halogenalkoxy, C₁-C₁₂-Halogenalkyl, C₄-C₁₄-Aryl, C₅-C₁₅-Arylalkyl oder Resten der Formel (V),

L-Q-T-W (V)

in der unabhängig voneinander
L fehlt oder für C₁-C₈-Alkylen oder C₂-C₈-Alkenylen steht und
Q fehlt oder für Sauerstoff, Schwefel oder NR¹¹ steht,
T für eine Carbonyl-Gruppe steht und
W für R¹¹, OR¹¹, NHR¹² oder N(R¹²)₂ steht,
wobei N(R¹²)₂ weiterhin als Ganzes für einen 5 oder 6 gliedrigen cyclischen Aminorest stehen kann oder Resten der Formeln (VIa-g)

| | | | |
|---|---|---|---|
| L-W | (VIa) | L-SO₂-W | (VIb) |
| L-NR¹²SO₂R¹² | (VIc) | L-SO₃Z | (VId) |
| L-P0₃Z₂ | (VIe) | L-COZ | (VIf) |
| L-CN | (VIg) | | |

in denen L, Q, W und R¹² die unter der Formel (IV) angegebene Bedeutung besitzen und Z für Wasserstoff oder M steht und in Formel (III)
- R⁶: für OR¹¹ oder N(R¹¹)₂ steht, wobei N(R¹¹)₂ zusammen auch für einen 5 oder 6 gliedrigen cyclischen Aminorest stehen kann,
- R⁷ und R⁸: jeweils unabhängig voneinander für Wasserstoff, C₁-C₈-Alkyl, C₄-C₁₀-Aryl, C₅-C₁₁-Arylalkyl oder C₂-C₈-Alkenyl stehen und
- R⁹ und R¹⁰: jeweils unabhängig voneinander für Wasserstoff, C₁-C₈-Alkyl, C₄-C₁₄-Aryl, C₅-C₁₁-Arylalkyl oder C₂-C₈-Alkenyl stehen oder
- R⁷ und R⁹ oder R⁸ und R¹⁰: jeweils zusammen für C₃-C₁₂-Alkylen oder C₃-C₁₂-Alkenylen stehen.

Die Verbindungen der Formel (I) können je nach Substitution auch chiral sein. Die Erfindung umfasst auch die gegebenenfalls auftretenden Stereoisomeren sowie beliebige Mischungen davon. Die Begriffe stereoisomerenangereichert (enantiomerenangereichert bzw. diastereomerenangereichert) bedeuten im Rahmen der Erfindung stereoisomerenreine (enantiomerenreine bzw. diastereomerenreine) Verbindungen oder Mischungen von Stereoisomeren (Enantiomeren bzw. Diastereomeren), in denen ein Stereoisomeres (Enantiomeres bzw. Diastereomeres) in einem größeren Anteil vorliegt als ein anderes bzw. das andere. Stereoisomerenangereichert bedeutet beispielsweise und bevorzugt einen Gehalt eines Stereoisomeren von 50 % bis 100 Gew.-%, besonders bevorzugt 70 % bis 100 Gew.-% und ganz besonders bevorzugt 90 bis 100 Gew.-%, bezogen auf die Summe der jeweiligen Stereoisomeren.

Der Rahmen der Erfindung umfasst alle Kombinationen der oben stehenden und im Folgenden aufgeführten, allgemeinen oder in Vorzugsbereichen genannten Restedefinitionen, Parameter und Erläuterungen untereinander, also auch beliebige Kombinationen zwischen den jeweiligen Bereichen und Vorzugsbereichen.

**Aryl** steht im Rahmen der Erfindung, sofern nicht gesondert angegeben, für carbocyclische aromatische Reste wie vorzugsweise Phenyl, Naphtyl, Phenanthrenyl und Anthracenyl, oder für heteroaromatische Reste, in denen keines, ein, zwei oder drei Gerüstkohlenstoffatome pro Cyclus, im gesamten Molekül mindestens jedoch ein Gerüstkohlenstoffatom durch Heteroatome substituiert sind, die ausgewählt sind aus der Gruppe Stickstoff, Schwefel oder Sauerstoff, wie vorzugsweise Pyridinyl, Oxazolyl, Thiophen-yl, Benzofuranyl, Benzothiophen-yl, Dibenzofuranyl, Dibenzothiophen-yl, Furanyl, Indolyl, Pyridazinyl, Pyrazinyl, Imidazolyl, Pyrimidinyl und Chinolinyl.

Weiterhin können die carbocyclischen, aromatischen Reste oder heteroaromatischen Reste mit bis zu fünf gleichen oder verschiedenen Substituenten pro Cyclus substituiert sein. Beispielsweise und bevorzugt sind die Substituenten ausgewählt aus der Gruppe Brom, Fluor, Chlor, Nitro, Cyano, freies oder geschütztes Formyl, freies oder geschütztes Hydroxy, C₁-C₁₂-Alkyl, C₁-C₁₂-Halogenalkyl, C₁-C₁₂-Alkoxy, C₁-C₁₂-Halogenalkoxy, C₄-C₁₄-Aryl wie zum Beispiel Phenyl, C₅-C₁₅-Arylalkyl wie zum Beispiel Benzyl, Di(C₁-C₁₂-alkyl)-amino, (C₁-C₁₂-Alkyl)-amino, CO(C₁-C₁₂-Alkyl), OCO(C₁-C₁₂-Alkyl), NHCO(C₁-C₁₂-Alkyl), N(C₁-C₈-Alkyl)CO(C₁-C₁₂-Alkyl), CO(C₄-C₁₄-Aryl), OCO(C₄-C₁₄-Aryl), NHCO(C₄-C₁₄-Aryl), N(C₁-C₈-Alkyl)CO(C₄-C₁₄-Aryl), COO-(C₁-C₁₂-Alkyl), COO-(C₄-C₁₄-Aryl), CON(C₁-C₁₂-Alkyl)₂ oder CONH(C₁-C₁₂-Alkyl) CO₂M, CONH₂, SO₂NH₂, SO₂N(C₁-C₁₂-Alkyl)₂, SO₃M wobei M jeweils für gegebenenfalls substituiertes Ammonium, Lithium, Natrium oder Kalium steht.

Beispielsweise und bevorzugt steht Aryl für Phenyl oder Naphthyl, das mit keinem, einem, zwei oder drei Resten pro Cyclus weiter substituiert sein kann, die ausgewählt sind aus der Gruppe Fluor, Chlor, Cyano, C₁-C₈-Alkyl, C₁-C₈-Perfluoralkyl, C₁-C₈-Alkoxy, Phenyl, Benzyl, Di(C₁-C₁₂-alkyl)-amino, CO(C₁-C₁₂-Alkyl), COO-(C₁-C₁₂-Alkyl), CON(C₁-C₁₂-Alkyl)₂ oder SO₂N(C₁-C₁₂-Alkyl)₂.

Besonders bevorzugt steht Aryl für Phenyl, das mit keinem, einem oder zwei Resten pro Cyclus weiter substituiert sein kann, die ausgewählt sind aus der Gruppe Fluor, Chlor, Cyano, C₁-C₄-Alkyl, C₁-C₄-Perfluoralkyl, C₁-C₄-Alkoxy, Phenyl oder SO₂N(C₁-C₄-Alkyl)₂.

Analog gelten die Definition und die Vorzugsbereiche im Rahmen der Erfindung auch für Aryloxy-Substituenten und den Arylteil eines Arylalkyl-Restes.

**Geschütztes Formyl** steht im Rahmen der Erfindung, sofern nicht gesondert angegeben, für einen Formyl-Rest, der durch Überführung in ein Aminal, Acetal oder ein gemischtes Aminalacetal geschützt ist, wobei die Aminale, Acetale und gemischten Aminalacetale acyclisch oder cyclisch sein können.

Beispielsweise und bevorzugt steht geschütztes Formyl für einen 1,1-(2,4-Dioxycyclopentandiyl)-Rest.

**Geschütztes Hydroxy** steht im Rahmen der Erfindung, sofern nicht gesondert angegeben, für einen Hydroxy-Rest, der durch Überführung in ein Ketal, Acetal oder ein gemischtes Aminalacetal geschützt ist, wobei die Acetale und gemischten Aminalacetale acyclisch oder cyclisch sein können.

Beispielsweise und bevorzugt steht geschütztes Hydroxy für einen Tetrahydropyranyl-Rest (O-THP).

**Alkyl** bzw. **Alkylen,** bzw. **Alkoxy** bzw. **Alkenyl** bzw. **Alkenylen** steht im Rahmen der Erfindung, sofern nicht gesondert angegeben, für einen geradkettigen, cyclischen, verzweigten oder unverzweigten Alkyl- bzw. Alkylen- bzw. Alkoxy- bzw. Alkenyl- bzw. Alkenylen-Rest, der gegebenenfalls durch C₁-C₄-Alkoxy in der Art weiter substituiert sein kann, dass jedes Kohlenstoffatom des Alkyl- bzw. Alkylen-, bzw. Alkoxy bzw. Alkenyl- bzw. Alkenylen-Restes höchstens ein Heteroatom ausgewählt aus der Gruppe Sauerstoff, Stickstoff oder Schwefel trägt.

Gleiches gilt analog für den Alkylenteil eines **Arylalkyl**-Restes.

Beispielsweise steht im Rahmen der Erfindung C₁-C₄-Alkyl bevorzugt für Methyl, Ethyl, 2-Ethoxyethyl, n-Propyl, Isopropyl, n-Butyl, tert.-Butyl, C₁-C₈-Alkyl darüber hinaus beispielsweise für n-Pentyl, Cyclohexyl, n-Hexyl, n-Heptyl, n-Octyl oder iso-Octyl, C₁-C₁₂-Alkyl, weiter darüber hinaus z.B. für Norbornyl, Adamantyl, n-Decyl und n-Dodecyl und C₁-C₁₈-Alkyl noch weiter darüber hinaus für n-Hexadecyl und n-Octadecyl.

Beispielsweise steht im Rahmen der Erfindung C₁-C₈-Alkylen bevorzugt für Methylen, 1,1-Ethylen, 1,2-Ethylen, 1,1-Propylen, 1,2-Propylen, 1,3-Propylen, 1,1-Butylen, 1,2-Butylen, 2,3-Butylen und 1,4-Butylen, 1,5-Pentylen, 1,6-Hexylen, 1,1-Cyclohexylen, 1,4-Cyclohexylen, 1,2-Cyclohexylen und 1,8-Octylen.

Beispielsweise steht im Rahmen der Erfmdung C₁-C₄-Alkoxy bevorzugt für Methoxy, Ethoxy, Isopropoxy, n-Propoxy, n-Butoxy und tert.-Butoxy, C₁-C₈-Alkoxy darüber hinaus für Cyclohexyloxy.

Beispielsweise steht im Rahmen der Erfindung C₂-C₈-Alkenyl bevorzugt für Allyl, 3-Propenyl und 4-Butenyl.

Beispielsweise steht im Rahmen der Erfindung C₃-C₈-Alkenylen bevorzugt für 2-Butendiyl.

**Halogenalkyl** bzw. **Halogenalkoxy,** steht im Rahmen der Erfindung, sofern nicht gesondert angegeben, für einen geradkettigen, cyclischen, verzweigten oder unverzweigten Alkyl- bzw. Alkoxy-Rest, der durch Halogenatome einfach, mehrfach oder vollständig substituiert ist. Reste die vollständig durch Fluor substituiert sind, werden mit **Perfluoralkyl** bzw. **Perfluoralkoxy** bezeichnet.

Beispielsweise steht im Rahmen der Erfindung C₁-C₁₂-Halogenalkyl für Trifluormethyl, 2,2,2-Trifluorethyl, Chlormethyl, Fluormethyl, Brommethyl, 2-Bromethyl, 2-Chlorethyl, Nonafluorbutyl, n-Perfluoroctyl oder n-Perfluordodecyl.

Im Folgenden werden die bevorzugten Substitutionsmuster für Verbindungen der Formel (I) definiert:
- R¹: steht bevorzugt entweder für einen Rest der Formel (II),
in der R² und R³ jeweils unabhängig voneinander für Wasserstoff, Fluor, Iod, C₁- C₁₂-Alkyl, C₁-C₁₂-Alkoxy, C₄-C₁₀-Aryl oder für Reste der Formel (IV) stehen, in der wiederum T für Carbonyl und Het für Sauerstoff oder NR¹¹ steht und in der
n und m jeweils unabhängig voneinander für 0 oder 1 stehen und
R⁴ und R⁵ jeweils unabhängig voneinander ausgewählt sind aus der Gruppe Fluor, Brom, Nitro, C₁-C₄-Alkyl, C₁-C₄-Alkoxy oder Resten der Formeln (VIb) und (VIg),
oder für einen Rest der Formel (III),
in der R⁶ für OR¹¹ oder N(R¹¹)₂ steht, wobei N(R¹¹)₂ zusammen auch für einen 5 oder 6 gliedrigen cyclischen Aminorest stehen kann, R⁷ und R⁸ jeweils unabhängig voneinander für Wasserstoff, C₁-C₄-Alkyl, oder C₂-C₈-Alkenyl stehen und R⁹ und R¹⁰ jeweils unabhängig voneinander für Wasserstoff, C₁-C₄-Alkyl, C₄- C₁₄-Aryl oder C₂-C₈-Alkenyl stehen, oder alternativ R⁸ und R¹⁰ jeweils zusammen für C₃-C₈-Alkendiyl stehen.
- R¹: steht besonders bevorzugt entweder für einen Rest der Formel (II),
in der R² und R³ jeweils unabhängig voneinander für Wasserstoff, C₁-C₁₂-Alkyl, C₁-C₁₂-Alkoxy, C₄-C₁₀-Aryl n und m jeweils identisch für 0 oder 1 stehen und
R⁴ und R⁵ jeweils identisch ausgewählt sind aus der Gruppe Fluor oder Resten der Formeln (VIb) und (VIg),
oder für einen Rest der Formel (III),
in der R⁶ für OR¹¹, R⁷ und R⁹ jeweils unabhängig voneinander für Wasserstoff, oder C₁-C₄-Alkyl und R⁸ und R¹⁰ jeweils zusammen für C₃-C₈-Alkendiyl stehen.
- R¹: steht ganz besonders bevorzugt entweder für einen Rest der Formel (II),
in der R² und R³ jeweils unabhängig voneinander für Wasserstoff, C₁-C₁₂-Alkyl, C₁-C₁₂-Alkoxy oder C₄-C₁₀-Aryl und
n und m jeweils für 0 stehen
oder für einen Rest der Formel (III),
in der R⁶ für OR¹¹, R⁷ und R⁹ jeweils identisch für Wasserstoff und R⁸ und R¹⁰ jeweils zusammen für C₃-C₈-Alkendiyl stehen.
- R² und R³: stehen bevorzugt jeweils unabhängig voneinander für Wasserstoff, Fluor, Iod, C₁- C₁₂-Alkyl, C₄-C₁₀-Aryl oder für Reste der Formel (IV), in der wiederum T für Carbonyl und Het für Sauerstoff oder NR¹¹ steht,
und stehen besonders bevorzugt jeweils unabhängig voneinander für Wasserstoff, C₁-C₁₂-Alkyl, C₁C₁₂-Alkoxy, C₄-C₁₀-Aryl.
- R⁴ und R⁵: sind bevorzugt jeweils unabhängig voneinander ausgewählt aus der Gruppe Fluor, Brom, Nitro, C₁-C₄-Alkyl, C₁-C₄-Alkoxy oder Resten der Formeln (VIb) und (VIg),
und besonders bevorzugt jeweils identisch ausgewählt aus der Gruppe Fluor oder Resten der Formeln (VIb) und (VIg),
- n und m: stehen bevorzugt jeweils unabhängig voneinander für 0 oder 1 stehen, n und m besonders bevorzugt jeweils identisch für 0 oder 1 stehen und ganz besonders bevorzugt jeweils für 0.
- R⁶: steht bevorzugt für OR¹¹ oder N(R¹¹)₂, wobei N(R¹¹)₂ zusammen auch für einen 5 oder 6 gliedrigen cyclischen Aminorest stehen kann, besonders bevorzugt für OR¹¹.

Bevorzugt stehen
R⁷ und R⁸ jeweils unabhängig voneinander für Wasserstoff, C₁-C₄-Alkyl, oder C₂-C₈-Alkenyl und
R⁹ und R¹⁰ jeweils unabhängig voneinander für Wasserstoff, C₁-C₄-Alkyl, C₄-C₁₄-Aryl oder C₂-C₈-Alkenyl, oder alternativ
R⁸ und R¹⁰ jeweils zusammen für C₃-C₈-Alkendiyl.

Besonders bevorzugt stehen
R⁷ und R⁹ jewells unabhängig voneinander für Wasserstoff oder C₁-C₄-Alkyl und
R⁸ und R¹⁰ jeweils zusammen für C₃-C₈-Alkendiyl.
Ganz besonders bevorzugt stehen
R⁷und R⁹ jeweils identisch für Wasserstoff und
R⁸und R¹⁰ jeweils zusammen für C₃-C₈-Alkendiyl.

Von Verbindungen der Formel (I) sind solche ganz besonders bevorzugt, die am Stickstoffatom Reste tragen, die ausgewählt sind aus der Gruppe
10-Cyano-5*H*-dibenzo[a,d]cyclohepten-5-yl (^{CN}trop), 5*H*-dibenzo[a,d]cydohepten-5-yl (trop), 10-Methyl-5*H* dibenzo[a,d]cyclohepten-5-yl (^{Me}trop),10-Methoxy-5*H*-dibenzo[a,d]cyclohepten-5-yl (^{MeO}trop), 10-Phenyl-*5H*-dibenzo[a,d]cyclohepten-5-yl (^{Ph}trop), 10,11-Diphenyl-*5H*-dibenzo[a,d]cyclohepten-5-yl(^{Ph2}trop) [(5S)-10-[(-)-Menthyloxy]-*5H*-dibenzo[a,d]cyclohepten-5-yl], (S-^{Menthyloxy}trop) und [(5R)-10-[(-)-Menthyloxy]-*5H*-dibenzo[a,d]cyclohepten-5-yl] (R-^{Menthyloxy}trop).

Zur Herstellung von Verbindungen der Formel (I) geht man vorzugsweise so vor, dass Verbindungen der Formel (IV)

H₂NR¹ (V)

in der R¹ die vorstehend genannte Bedeutung besitzt
gegebenenfalls in Gegenwart von organischem Lösungsmittel mit Verbindungen der Formel (V) umgesetzt werden, 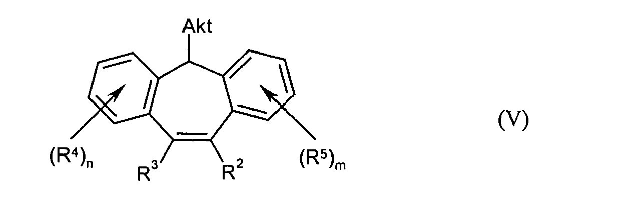 in der R², R³, R⁴, R⁵ , n und m die vorstehend genannten Bedeutungen besitzen und
Akt für Chlor, Brom, Iod, Trifluoracetyl oder einen Sulfonyloxy-Rest steht,
die dabei entstehenden Ammoniumsalze der Formel (VI) 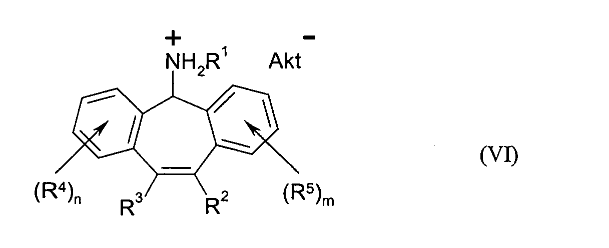 in Gegenwart von Base in Verbindungen der Formel (I) überführt werden.

Die Verbindungen der Formel (IV) und (V) sind entweder literaturbekannt oder analog zur Literatur synthetisierbar.

Die Umsetzung kann gegebenenfalls und wird bevorzugt in Gegenwart von organischem Lösungsmittel durchgeführt. Als organische Lösungsmittel sind beispielsweise geeignet:

Aliphatische oder aromatische, gegebenenfalls halogenierte Kohlenwasserstoffe, wie beispielsweise verschiedene Benzine, Benzol, Toluol, Xylol, Chlorbenzol, Dichlorbenzol, verschiedene Petrolether, Hexan, Cyclohexan, Dichlormethan, Chloroform, Tetrachlorkohlenstoff; Ether, wie Diethylether, Methyl-tert.-butylether, Diisopropylether, Dioxan, Tetrahydrofuran oder Ethylenglykoldimethyl- oder -diethylether oder Gemische solcher organischen Lösungsmittel.

Als Basen sind beispielsweise geeignet: Erdalkali- oder Alkalimetallhydride, -hydroxide, -amide, alkylsubstituierte -disilylamide, -dialkylamide, -alkoholate, oder -carbonate wie beispielsweise Natriumhydrid, Natriumamid, Lithium-diethylamid, Natriummethylat, Natriumbistrimethylsilylamid, Natriumethylat, Kalium-tert.-butylat, Natriumhydroxid, Kaliumhydroxid, Natriumcarbonat und Kaliumcarbonat, tertiäre Amine, wie Trimethylamin, Triethylamin, Tributylamin, Trioctylamin, Di-isopropyl-ethylamin, Tetramethylguanidin, N,N-Dimethylanilin, Diazabicyclooctan (DABCO), Diazabicyclononen (DBN) oder Diazabicycloundecen (DBU), Piperidin und N-Methylpiperidin.

Sowohl das Herstellungsverfahren für die Verbindungen der Formel (I) als auch die Verbindungen der Formel (VI) als unverzichtbare Intermediate sind von der Erfindung vollumfänglich umfasst.

Die Erfindung schließt auch Übergangsmetallkomplexe von Verbindungen der Formel (I) ein sowie Katalysatoren ein, die die erfindungsgemäßen Übergangsmetallkomplexe von Verbindungen der Formel (I) enthalten.

Bevorzugte Übergangsmetallkomplexe sind Übergangsmetallkomplexe von Ruthenium, Osmium, Cobalt, Rhodium, Iridium, Nickel, Palladium, Platin und Kupfer, bevorzugt solche von Ruthenium, Rhodium, Iridium, Nickel, Palladium und Platin, besonders bevorzugt solche von Rhodium und Iridium.

Als Katalysatoren können beispielsweise isolierte Übergangsmetallkomplexe eingesetzt werden, die beispielsweise aus den Verbindungen der Formel (I) und einer Metallverbindung erzeugt wurden, oder Übergangsmetallkomplexe, die aus den Verbindungen der Formel (I) und einer Metallverbindung im Reaktionsmedium der Katalyse erzeugt werden.

Geeignete Metallverbindungen sind beispielsweise und bevorzugt solche der Formel (VIIa)

M¹(Y¹)ₚ (VIIa)

in der
- M¹: für Ruthenium, Rhodium, Iridium, Nickel, Palladium, Platin oder Kupfer und
- Y¹: für Chlorid, Bromid, Acetat, Nitrat, Methansulfonat, Trifluormethansulfonat oder Acetylacetonat und
- p: im Falle von Ruthenium, Rhodium und Iridium für 3, im Falle von Nickel, Palladium und Platin für 2 und im Falle von Kupfer für 1 steht,
oder Metallverbindungen der allgemeinen Formel (VIIb)

M¹(Y²)ₚB¹ ₂ (VIIb)

in der
- Y²: für ein Anion wie beispielsweise Chlorid, Bromid, Acetat, Methansulfonat, Trifluormethansulfonat, Tetrafluoroborat, Hexafluorophosphat, Perchlorat, Hexafluoroantimonat, Tetra(bis-3,5-trifluormethylphenyl)borat oder Tetraphenylborat steht und
- p: im Falle von Rhodium und Iridium für 1, im Falle von Nickel, Palladium, Platin und Ruthenium für 2 und im Falle von Kupfer für 1 steht,
- B¹: jeweils für ein C₂-C₁₂-Alken wie beispielsweise Ethylen oder Cycloocten, oder ein Nitril wie beispielsweise Acetonitril, Benzonitril oder Benzylnitril steht, oder
- B¹₂: zusammen für ein (C₄-C₁₂)-Dien wie beispielsweise Norbornadien oder 1,5-Cyclooctadien steht
oder Metallverbindungen der Formel (VIIc)

[M²B²Y¹ ₂]₂ (VIIc)

in der
- M²: für Ruthenium und
- B²: für Arylreste wie zum Beispiel Cymol, Mesityl, Phenyl oder Cyclooctadien, Norbornadien oder Methylallyl steht
oder Metallverbindungen der Formel (VIId)

Meₚ[M³(Y³)₄] (VIId),

wobei
- M³: für Palladium, Nickel, Iridium oder Rhodium und
- Y³: für Chlorid oder Bromid steht und
- Me: für Lithium, Natrium, Kalium, Ammonium oder organisches Ammonium steht und
- p: im Falle von Rhodium und Iridium 3, im Falle von Nickel, Palladium und Platin für 2 steht,
oder Metallverbindungen der Formel (VIIe)

[M⁴(B³)₂]An (VIIe),

wobei
- M⁴: für Iridium oder Rhodium und
- B³: für ein (C₄-C₁₂)-Dien wie beispielsweise Norbornadien oder 1,5-Cyclooctadien steht
- An: für ein nicht oder schwach koordinierendes Anion wie zum Beispiel Methansulfonat, Trifluormethansulfonat, Tetrafluoroborat, Hexafluorophosphat, Perchlorat, Hexafluoroantimonat, Tetra(bis-3,5-trifluormethylphenyl)borat oder Tetraphenylborat steht.

Darüberhinaus sind als Metallverbindungen beispielsweise Ni(1,5-Cyclooctadien)₂, Pd₂(dibenzylidenaceton)₃, Pd[PPh₃]₄ Cyclopentadienyl₂Ru, Rh(acac)(CO)₂, [RhCl(CO)₂]; Ir(pyridin)₂(1,5-Cyclooctadien), Ir(acac)(CO)₂, [IrCl(CO)₂], Cu(Phenyl)Br, Cu(Phenyl)Cl, Cu(Phenyl)I, Cu(PPh₃)₂Br, [Cu(CH₃CN)₄]BF₄ und [Cu(CH₃CN)₄]PF₆ oder mehrkernige verbrückte Komplexe wie beispielsweise [Rh(1,5-cyclooctadien)Cl]₂ und [Rh(1,5-cyclooctadien)Br]₂, [Rh(Ethen)₂Cl]₂, [Rh(Cycloocten)₂Cl]₂ geeignet.

Bevorzugt werden als Metallverbindungen eingesetzt:

[Rh(COD)Cl]₂, [Rh(COD)₂Br], [Rh(COD)₂]ClO₄, [Rh(COD)₂]BF₄, [Rh(COD)₂]PF₆, [Rh(COD)₂]OTf, [Rh(COD)₂]BAr₄ (Ar = 3,5-bistrifluormethylphenyl) [Rh(COD)₂]SbF₆ RuCl₂(COD), [(Cymol)RuCl₂]₂, [(Benzol)RuCl₂]₂, [Mesitylen)RuCl₂]₂, [(Cymol)RuBr₂]₂, [(Cymol)RuI₂]₂, [(Cymol)Ru(BF₄)₂]₂, [(Cymol)Ru(PF₆)₂]₂, [(Cymol)Ru(BAr₄)₂]₂, (Ar = 3,5-bistrifluormethylphenyl), [(Cymol)Ru(SbF₆)₂]₂, [Ir(COD)₂Cl]₂, [Ir(COD)₂]PF₆, [Ir(COD)₂]ClO₄, [Ir(COD)₂]SbF₆ [Ir(COD)₂]BF₄, [Ir(COD)₂]OTf, [Ir(COD)₂]BAr₄ (Ar = 3,5-bistrifluormethylphenyl), RuCl₃, NiCl₂, RhCl₃, PdCl₂, PdBr₂, Pd(OAc)₂, Pd₂(dibenzylidenaceton)₃, Pd(acetylacetonat)₂, Rh(acetylacetonat)(CO)₂, [RhCl(CO)₂]; Ir(pyridin)₂(COD), Ir(acac)(CO)_{2'} [IrCl(CO)₂] [Rh(nbd)Cl]₂, [Rh(nbd)₂Br], [Rh(nbd)₂]ClO₄, [Rh(nbd)₂]BF₄, [Rh(nbd)₂]PF₆, [Rh(nbd)₂]OTf, [Rh(nbd)₂]BAr₄ (Ar = 3,5-bistrifluormethylphenyl) [Rh(nbd)₂]SbF₆ RuCl₂(nbd), [Ir(nbd)₂]PF₆, [Ir(nbd)₂]ClO₄, [Ir(nbd)₂]SbF₆ [Ir(nbd)₂]BF₄, [Ir(nbd)₂]OTf, [Ir(nbd)₂]BAr₄ (Ar = 3,5-bistrifluormethylphenyl), Ir(pyridin)₂(nbd), [Ru(DMSO)₄Cl₂], [Ru(CH₃CN)₄Cl₂], [Ru(PhCN)₄Cl₂], [Ru(COD)Cl₂]ₙ, [Ru(COD)(Methallyl)₂], [Ru(acetylacetonat)₃]

Noch weiter bevorzugt sind Rh(acetylacetonat)(CO)₂, [RhCl(CO)₂] und Ir(acetylacetonat)(CO)₂, [IrCl(CO)₂].

Die Menge der eingesetzten Metallverbindung kann bezogen auf den Metallgehalt beispielsweise 25 bis 200 mol-% im Bezug auf die eingesetzte Verbindung der Formel (I) betragen, bevorzugt sind 80 bis 140 mol-%, ganz besonders bevorzugt 90 bis 120 mol-% und noch weiter bevorzugt 95 bis 105 mol% .

Ganz besonders bevorzugte Übergangsmetallkomplexe von Verbindungen der Formel (I) sind solche die der Formel (VIII) gehorchen

[M⁵(I)(L)]An (VIII)

in denen jeweils
- M⁵: für Rhodium oder Iridium
- (I): für eine Verbindung der Formel (I)
- (L): für einen neutralen mono- oder bidentanten Liganden steht und
- An: ohne Rücksicht auf eine mögliche Koordination an das Metall für ein Anion wie beispielsweise Chlorid, Bromid, Iodid, Methansulfonat, Trifluormethansulfonat, Tetrafluoroborat, Hexafluorophosphat, Perchlorat, Hexafluoroantimonat, Tetra(bis-3,5-trifluormethylphenyl)borat oder Tetraphenylborat steht.

Bevorzugte neutrale mono-dentante Liganden sind beispielsweise Olefine wie Cyclohexen, Carbonyl, Nitrile wie beispielsweise Acetonitril oder Benzonitril, Phosphane wie beispielsweise Tri-(C₄-C₁₄-Aryl)phosphane, Tri-(C₁-C₈-alkyl)phosphane, Bis-(C₄-C₁₄-aryl)(C₁-C₈-alkyl)phosphane, (C₄-C₁₄-Aryl)-di(C₁-C₈-alkyl)phosphane, Phosphite wie beispielsweise Tri-(C₄-C₁₄-Aryl)phosphite, Tri-(C₁-C₈-alkyl)phosphite, Bis-(C₄-C₁₄-aryl)(C₁C₉-alkyl)phosphite und (C₄-C₁₄-Aryl)-di(C₁-C₈-alkyl)phosphite.

Bevorzugte neutrale bidentante Liganden sind beispielsweise Diolefine wie 1,5-Cyclooctadien, Norbornadien, Distickstoffverbindungen wie beispielsweise 2,2'-Bipyridin und Diphosphorverbindungen wie beispielsweise solche der Formel (IX),

(R¹³-E)₂P-E-Z-E-P(E-R¹³)₂ (IX)

in der
- E: jeweils unabhängig voneinander und unabhängig von R¹³ und Z fehlt oder für Sauerstoff steht und die Reste R¹³ unabhängig voneinander für C₁-C₈-Alkyl oder für unsubstituiertes, ein-, zwei oder dreifach durch R¹⁴ substituiertes Phenyl- , Naphtyl- oder Heteroaryl mit 5 bis 12 Gerüstkohlenstoffatomen stehen, wobei
R¹⁴ jeweils unabhängig ausgewählt ist aus der Gruppe C₁-C₈-Alkyl, C₁-C₈-Alkoxy, Fluor oder Cyano und
- Z: für einen unsubstituierten oder substituierten Rest aus der Gruppe C₁-C₄-Alkylen, 1,2-Phenylen, 1,3-Phenylen, 1,2-Cyclohexyl, 1,1'-Ferrocenyl, 1,2-Ferrocenyl, 2,2'-(1,1'-Binaphtyl) und 1,1'-Biphenylyl steht.

Katalysatoren, die die erfindungsgemäßen Übergangsmetallkomplexe enthalten, eignen sich insbesondere für den Einsatz in der homogenen Katalyse.

Den genannten Übergangsmetallkomplexen ist gemeinsam, dass das Proton, das am Stickstoffatom des zentralen Siebenringes der Verbindung der Formel (I) gebunden ist, sehr azide ist und sich durch Base leicht entfernen lässt.

Solche Übergangsmetallkomplexe sind für den Einsatz als Katalysator ebenfalls geeignet, und stellen nach Erkenntnis des Anmelders auch Intermediate des katalytischen Cyclus dar.

Von der Erfindung sind daher weiterhin Übergangsmetallkomplexe umfasst, die die Verbindungen der Formel (I) in der Art enthalten, dass das Stickstoffatom des zentralen Siebenringes von Verbindungen der Formel (I) als Amid an das Metallatom koordiniert.

Für solche Komplexe gelten oben genannte Vorzugsbereiche für die Verbindungen der Formel (I) und die Übergangsmetallkomplexe in analoger Weise.

Besonders bevorzugte Übergangsmetallkomplexe dieser Art sind dementsprechend solche der Formel (X)

[M⁶(I-)(L)] (X)

in denen jeweils
- M⁶: für Rhodium oder Iridium
- (I-): für eine Verbindung der Formel (I) steht, in der das Stickstoffatom des zentralen Siebenringes als Amid an das Metallatom koordiniert und
- (L): für einen neutralen mono- oder bidentanten Liganden steht.

Bevorzugt werden die erfindungsgemäßen Katalysatoren und Übergangsmetallkomplexe für Hydrogenierungen eingesetzt, besonders bevorzugt für asymmetrische Hydrogenierungen, sofern die Verbindungen unter eingangs beschriebenen Voraussetzungen chiral sind.

Bevorzugte Hydrogenierungen sind beispielsweise Hydrogenierungen von prochiralen C=C-Bindungen wie zum Beispiel prochiralen Enaminen, Olefinen, Enolethern, C=O-Bindungen wie zum Beispiel prochiralen Ketonen und C=N-Bindungen wie zum Beispiel prochiralen Iminen. Besonders bevorzugte asymmetrische Hydrogenierungen sind Hydrogenierungen von C=O-Bindungen wie zum Beispiel prochiralen Ketonen.

In einer bevorzugten Ausführungsform wird die Hydrogenierung in Gegenwart eines Wasserstoffspendermoleküls und gegebenenfalls einer Base durchgeführt.

Wasserstoffspendermoleküle sind beispielsweise molekularer Wasserstoff, Ameisensäure, Ethanol oder Isopropanol, Basen beispielsweise Alkoholate oder tertiäre Amine. Besonders bevorzugte Mischungen von Wasserstoffspendermolekül und Base sind Mischungen von Ameisensäure und Triethylamin wie insbesondere das azeotrope Gemisch daraus, sowie Mischungen von Kaliumisopropylat und Isopropanol.

Die Menge der eingesetzten Metallverbindung oder des eingesetzten Übergangsmetallkomplexes kann bezogen auf den jeweiligen Metallgehalt beispielsweise 0.001 bis 20 mol-%, bezogen auf das eingesetzte Substrat betragen, bevorzugt sind 0.001 bis 2 mol-%, ganz besonders bevorzugt 0.001 bis 1 mol-%.

In einer bevorzugten Ausführungsform können asymmetrische Hydrogenierungen beispielsweise so durchgeführt werden, dass der Katalysator in situ aus einer Metallverbindung und einer Verbindung der Formel (I) gegebenenfalls in einem geeigneten organischen Lösungsmittel erzeugt wird, das Substrat zugegeben wird und die Reaktionsmischung bei Reaktionstemperatur entweder unter Wasserstoffdruck gesetzt oder mit einer Mischung aus einem anderen Wasserstoffspendermolekül und einer Base versetzt wird.

Die erfindungsgemäßen Katalysatoren eignen sich insbesondere in einem Verfahren zur Herstellung von Wirkstoffen von Arzneimitteln und Agrarchemikalien, oder Zwischenprodukten dieser beiden Klassen.

Der Vorteil der vorliegenden Erfindung liegt darin, dass unter Verwendung von einfach erhältlichen und gefahrlos handhabbaren Verbindungen eine ganze Klasse leistungsfähiger Katalysatoren hergestellt werden können.

### Beispiele

### Beispiel 1: Synthese von Bis-(5H-dibenzo[a,d]cyclohepten-5-yl)-amin [trop₂NH]

3 g *5H-*Dibenzo[a,d]cyclohepten-5-yl)chlorid (13.2 mmol) wurden in 60 ml Toluol gelöst und mit 1 ml (1.41 g; 8.7 mmol; 30% Überschuss) 1,1,1,3,3,3-Hexamethyl-disilazan versetzt. Die Mischung wurde 4 h unter Rückfluss erhitzt. Anschließend wurden alle flüchtigen Bestandteile im Vakuum entfernt. Anschließend wurden 30 ml Hexan zugesetzt und die entstehende Suspension erhitzt. Dabei fiel ein weißes Pulver mit einer Ausbeute von 95 % d. Th. an.

### Beispiel 2: Synthese von [Rh{(trop)₂NH}Cl]₂

In 7 ml Dichlormethan wurden 0.47 g (1.2 mmol) Bis(*5H*-dibenzo[*a*,*d*]cyclohepten-5-yl)amin gemäß Beispiel 1 und 0.28 g (0.56 mmol) Di-µ-chloro-bis[(η-1,5-cyclooctadien)rhodium(I)] gelöst und zwei Tage stehen gelassen. Dabei wuchsen dunkelrote Kristall. Das überstehende Lösungsmittel wurde dekantiert. Die Kristalle wurden mit Dichlormethan gewaschen und im Vakuum getrocknet.

### Beispiel 3: Synthese von [Rh{(trop)₂NH}(CO)Cl] (3a) und [Rh{(trop)₂NH}(CO)]OTf(3b)

0.63 g (0.59 mmol) Di-µ-chloro-bis[(bis(*5H*-dibenzo[*a*,*d*]cyclohepten-5-yl)amin)-rhodium(I)] aus Beispiel 2 wurden in THF suspendiert. Die Luft wurde aus dem Kolben entfernt. Daraufhin wurde Kohlenmonoxid durch die Suspension geleitet, worauf die trübe orange Lösung klar und gelb wurde. Dabei wurde 3a erhalten Nach Zugabe von 0.31 g (1.2 mmol) Silbertriflat wurde die Lösung rot. Nach 24 Stunden wurde der Silberchloridniederschlag abfiltriert, die Lösung zum Teil eingeengt und der Komplex 3b als oranges Pulver mit Hexan ausgefällt. Ausbeute: 0.58 g (86 %).

### Beispiel 4: Synthese von [Rh{(trop)₂NH}(PPh₃)Cl]

0.16 g (0.15 mmol) Di-µ-chloro-bis[(bis(5H-dibenzo[a,d]cyclohepten-5-yl)amin)rhodium(I)] und 0.09 g (0.3 mmol) Triphenylphosphin wurden in 20 ml THF suspendiert. Die Suspension wurde bei 60°C eine klare Lösung. Es wurden ca. 30 ml Toluol und 100 ml Hexan zugegeben, worauf (Bis(*5H*-dibenzo[*a*,*d*]cyclohepten-5-yl)amin)chloro-(triphenylphosphin)-rhodium(I) ausfiel.
Dieses konnte abfiltriert werden. Ausbeute: 0.17 g (70 %)
¹H NMR (250 MHz, CDCl₃) δ = 0.4 (s b, 1H, amin) 3.8 (s, 2 H, benzyl) 5.3 (t, *J*=7.5 Hz, 2 H, olefin) 5.4 (t, *J*=8.5 Hz, 2 H, olefin) 5.6 (ddd, *J*=9.4, 5.7, 1.5 Hz, 2 H, phenyl) 6.5 (d, *J*=6.8 Hz, 2 H, arom) 6.6-7.3 (m, 17 H, arom) 7.4 (d, *J*=5.3 Hz, 6 H, arom) 8.1 (m, 4 H, phenyl) ³¹P NMR (101 MHz) δ = 7.7 (d, *J*=110.9 Hz, 1 P)

### Beispiel 5: Synthese von [Rh{(trop)₂NH}(PPh₃)]OTf

0.17 g (0.21 mmol) (Bis(*5H*-dibenzo[*a*,*d*]cyclohepten-5-yl)amin)chloro-(triphenyl-phosphin)-rhodium(I) aus Beispiel 4 und 0.06 g (0.3 mmol) Silbertriflat wurden bei 60°C eine halbe Stunde gerührt. Die Suspension wurde über einen Papierfilter filtriert. Das Produkt wurde mit Hexan aus der Mutterlauge ausgefällt. Ausbeute: 0.147 g (70 %).
¹H NMR (250 MHz, CDCl₃) δ = 5.0 (d, *J*=8.7 Hz, 4 H, 2 olefin, 2 benzyl) 5.5 (td, *J*=9.4, 3.0 Hz, 2 H, olefin) 5.7 (d, *J*=5.3 Hz, 1 H, amin) 6.8 (m, 8 H, arom) 7.3 (m, 8 H, arom) 7.6 (s, 9 H, m u. p phenyl) 7.9 (m, 6 H, o phenyl)
³¹P NMR (101 MHz, CDCl₃) δ = 38.8 (d, *J*=138.0 Hz, 1 P)

### Beispiel 6: Synthese von [Rh{(trop)₂NH}(P(OCH₃)₃]OTf

500 mg des Komplexes aus Beispiel 3b (0,74 mmol) wurden in 60 ml THF gelöst und mit 0,5 ml Trimethylphosphit versetzt. Die Farbe der Lösung schlug dabei von Gelb nach blassgelb um und es fielen nach 2 Stunden gelbe Nadeln des gewünschten Produktes aus. Ausbeute: 99 % d. Th.
¹H NMR (400.1 MHz, CD₂Cl₂) δ = 3.34 (d, ³*J*_{PH} = 10.5 Hz, 9 H, H¹²), 3.99 (d, ³*J*_{HH}= 10.7 Hz, 9 H, H¹³), 4.17 (d, ³*J*_{PH} = 5.7 Hz, 1 H, N*H*), 4.83 (d, ⁴*J*_{PH} = 13.0 Hz, 2 H, H⁵), 5.15 (m, 2 H, H¹¹), 5.17 (m, 2 H, H¹⁰), 6.90 (d, ³*J*_{HH} = 7.8 Hz, 2 H, H⁴), 6.91 (dd, ³*J*_{HH} = 7.7 Hz, ³*J*_{HH} = 7.7 Hz, 2 H, H²), 6.97 (m, 2 H, H³), 6.99 (m, 2 H, H¹), 7.20 (ddd, ³*J*_{HH} = 7.4 Hz, ³*J*_{HH} = 7.4 Hz, ⁴*J*_{HH} = 1.2 Hz, 2 H, H⁷), 7.28 (ddd, ³*J*_{HH} *=* 7.5 Hz, ³*J*_{HH} = 7.5 Hz,⁴*J*_{HH} = 1.4 Hz, 2 H, H⁸), 7.30 (dd, ³*J*_{HH} = 7.4 Hz, ⁴*J*_{HH} = 1.4 Hz, 2 H, H⁶), 7.49 (dd, ³*J*_{HH} = 7.53 Hz, ⁴*J*_{HH} = 1.1 Hz, 2 H, H⁹)
³¹P NMR (162.0 MHz, CD₂Cl₂) δ = 112.1 (dd, ¹*J*_{*RhP*} *=* 190 Hz, ²*J*_{*PP*} *=* 68 Hz, *P*ₐₓᵢₐₗ), 124.5 (dd, ¹*J*_{*RhP*} = 200 Hz, ²*J*_{*PP*} *=* 68 Hz, *P*_{equat})

### Beispiel 7: Synthese von [Rh{(trop)₂N-}(PPh₃]

200 mg des Komplexes aus Beispiel 5 wurden in 50 ml THF suspendiert und mit 30 mg (0,267 mmol) KOtBu versetzt. Die Farbe der Lösung schlug dabei nach grün und homogenisierte sich innerhalb von 30 Minuten. Nach Filtration des ausgefallenen KOTf und Trocknen im Vakuum wurde das gewünsche Produkt in einer nahezu quantitativen Ausbeute erhalten.
¹H NMR (400.1 MHz, THF-d8, 200 K) δ = 4.69 (ddd, ³*J*_{HH} = 9.0 Hz, ³*J*_{PH} = 6.2 Hz, ²*J*_{RhH} = 1.2 Hz, 2 H, H¹⁰), 4.92 (d, ⁴*J*_{PH} = 13.5 Hz, 2 H, H⁵), 5.62 (ddd, ³*J*_{HH} = 9.0 Hz, ²*J*_{RhH} = 3.3 Hz, ³*J*_{PH} = 2.9 Hz, 2 H, H¹¹), 6.57 (dd, ³*J*_{HH} = 7.3 Hz, ³*J*_{HH} = 7.3 Hz, 2 H, H²), 6.67 (dd, ³*J*_{HH} = 7.2 Hz, ³*J*_{HH} = 7.2 Hz, 2 H, H³), 6.79 (d, ³*J*_{HH} =7.6 Hz, 2 H, H¹), 6.90 (d, ³*J*_{HH} = 7.3 Hz, 2 H, H⁴), 6.95 (d, ³*J*_{HH} = 7.0 Hz, 2 H, H⁹), 7.03 (m, 4 H, H⁷/ H⁸), 7.22 (d, ³*J*_{HH} =6.7 Hz, 2 H, H⁶), 7.56 (m, 9 H, m-PPh₃/ p-PPh₃), 7.63 (m, 6 H, o-PPh₃)

### Beispiel 8: Hydrogenierungen mit [Rh{(trop)₂N-}(PPh₃] aus Beispiel 7

Eine Lösung von 5 mg (0.0066 mmol) des Komplexes aus Beispiel 7 in 5 ml THF wurde mit 129 mg Cyclohexanon (1.31 mmol, 200 eq.) versetzt und während 18 h unter einer Wasserstoffatmosphäre (4 bar) gerührt. Gaschromatographie der Reaktionslösung zeigte vollständigen Umsatz zu Cyclohexanol.

### Beispiele 9 bis 23: Hydrogenierungen mit den Komplexen aus den Beispielen 3b, 5 und 6

| | Katalysator aus Beispiel | Substrat | C/S^{a}ppm | Alkoho 1 | TON^{b} | TOFᵢ^{c}[1/h] | TOFₜ^{d} [1/h] | tₘₐₓ^{e} [h] (Uₘₐₓ [%]) |
|---|---|---|---|---|---|---|---|---|
| 9 | 3b | Cyclohexanon | 1000 | *i*PrOH | 950 | 1700 | 640 | 1.5 (97) |
| 10 | 3b | Cyclohexanon | 250 | *i*PrOH | 3900 | 990 | 420 | 9.2 (96) |
| 11 | 3b | Cyclohexanon | 730 | *i*PrOH | 1400 | 1300 | 370 | 3.7 (98) |
| 12 | 3b | Cyclohexanon | 250 | EtOH | 3800 | 3300 | 1900 | 2.0 (100) |
| 13 | 3b | Acetophenon | 980 | *i*PrOH | 760 | 530 | 28 | 27.0 (74) |
| 14 | 3b | Benzophenon | 990 | *i*PrOH | 360 | 850 | 40 | 9.0 (35) |
| 15 | 3b | Benzophenon | 4000 | *i*PrOH | 290 | 1200 | 35 | 9.2 (61) |
| 16 | 6 | Cyclohexanon | 980 | *i*PrOH | 1000 | - | 4100 | <0.25 (100) |
| 17 | 6 | Cyclohexanon | 63 | *i*PrOH | 15300 | 23900 | 46000 | 0.3(97) |
| 18 | 6 | Cyclohexanon | 4.4 | *i*PrOH | 18100 | 15400 | 120000 | 3.7 (79) |
| 19 | 6 | Benzophenon | 1000 | *i*PrOH | 940 | 9500 | 1100 | 0.8 (97) |
| 20 | 5 | Cyclohexanon | 1000 | *i*PrOH | 1000 | - | 4000 | <0.25 (100) |
| 21 | 5 | Cyclohexanon | 49 | *i*PrOH | 20300 | 126000 | 120000 | 0.18 (100) |
| 22 | 5 | Cyclohexanon | 49 | EtOH | 20250 | 22000 | 81000 | <0.25 (100) |
| 23 | 5 | Acetophenon | 49 | EtOH | 20100 | 79000 | 60000 | 0.3 (100) |

| | | | | | | | | |
|---|---|---|---|---|---|---|---|---|
| a) C/S = (Stoffmenge an Katalysator)* 10⁶/(Stoffmenge an Substrat), | | | | | | | | |
| b) TON = (Stoffmenge an Produkt)/(Stoffmenge an Katalysator) am Ende der Katalyse angegeben, beziehungsweise zum Zeitpunkt, an welchem die Reaktion abgebrochen wurde, | | | | | | | | |
| c) TOF am Anfang der Katalyse, | | | | | | | | |
| d) TOF am Ende der Katalyse, | | | | | | | | |
| e) Zeit, nach welcher maximaler Umsatz erreicht war. Der maximal erreichte Umsatz steht in Klammern dahinter. | | | | | | | | |

### Beispiel 24: Synthese von N-(5H-dibenzo[a,d]cyclohepten-5-yl)-L-2,5-cyclohexadienylalaninmethylester (trop-cyclohexadienylalanin)

Zu 0.915 g (4.2 mmol) Cyclohexadienylalaninmethylester in 10 ml trockenem CH₂Cl₂ wurden 0.42 g (4.2 mmol) Triethylamin tropfenweise zugegeben. Nach Rühren für 30 Minuten wurden 0.952 g (4.2 mmol) 5*H*-Dibenzo[a,d]cyclohepten-5-yl-chlorid über einen Pulvertrichter zugegeben und weitere 2,0 g Triethylamin in einer Portion zugegeben. Nach weiteren 2 h Rühren wurde die organische Phase mit 2x10 ml Wasser gewaschen, über MgSO₄ getrocknet und filtriert, Nach Abziehen des Lösungsmittels im Vakuum wurden 1.52 g (97%) des Produktes als farbloses Öl erhalten.
¹H-NMR (300 MHz, 25°C, CDCl₃): δ 7.85 - 7.30 (m, 8H), 7.08 (dd, 2H), 5.87-5.74 (m, 2H), 5.50 (s, 1H), 5.00 (s, 1H), 3.85, 3.74 (each s, together 3H), 3.11 (dd, 1H), 2.90-2.00 (m, 7H) ppm.

### Beispiel 25: Synthese von [Rh(trop-cyclohexadienylalanin)(CO)Cl]

Zu einer Lösung von 220 mg (0.59 mmol) des Liganden aus Beispiel 24 in 10 ml trockenem CH₂Cl₂ wurden 97 mg (0.25 mmol) [Rh(CO)₂Cl]₂ bei Raumtemperatur zugegeben. Dabei wurde starke Entwicklung von Kohlenmonoxid beobachtet. Die Lösung wurde auf etwa 3 ml eingeengt und mit 10 ml Hexan überschichtet. Über Nacht kristallisierten 245 mg (92%) des Komplexes in Form fahlgelber Kristalle.
¹H-NMR (300 MHz, 25°C, CDCl₃): δ 7.60 - 7.05 (m, 8H), 5.77 (dm, ³J (H,H) = 9.9 Hz, 1H), 5.64 (dm, ³J (H,H) = 9.9 Hz, 1H), 5.24 (dd, ³J (H,H) = 9.3 Hz, ²J (Rh,H) = 1.2 Hz, 1H), 5.15 (dd, ³J (H,H) = 9.3 Hz, ²J (Rh,H) = 2.4 Hz, 1H), 4.43 (br, 1H), 4.39 (s, 1H), 3.84 (br, 1H), 3.79 (s, 3H), 3.45 (m, 1 H), 2.92 - 2.38 (m, 6H) ppm.

### Beispiel 26: Synthese von [Rh(trop-cyclohexadienylalanin)(CO)]OTf

Zu einer Lösung von 119 mg (0.22 mmol) des Komplexes aus Beispiel 25 in 10 ml trockenem CH₂Cl₂ wurden 64 mg (0.25 mmol) Silbertriflat bei Raumtemperatur zugegeben. Nach zweistündigem Rühren wurde die Lösung über Celite filtriert, auf etwa 3 ml eingeengt und mit 10 ml Hexan überschichtet. Über Nacht kristallisierte das Produkt in nahezu quantitativer Ausbeute.
¹H-NMR (300 MHz, 25°C, CDCl₃): δ 7.68 - 7.22 (m, 8H), 5.91 (dm, ³J (H,H) = 10.5 Hz, 1H), 5.75 (dm, ³J (H,H) = 10.5 Hz, 1H), 5.68 (dd, ³J (H,H) = 9.0 Hz, ²J (Rh,H) = 3.0 Hz, 1H), 5.45 (dd, ³J (H,H) = 9.0 Hz, ²J (Rh,H) = 0.9 Hz, 1H), 4.64 (br, 1H), 4.47 (br, 1H), 4.43 (br, 1H), 3.87 (s, 3H), 3.38 - 2.50 (m, 7H) ppm.

### Beispiele 27 und 28: Hydrogenierungen mit [[Rh{trop-cyclohexydienylalanin}(CO)]OTf aus Beispiel 26

| **Katalysatormenge [mol-%]** | **Temp. [°C]** | **Zeit [min]** | **Substrat** | **Umsatz** | **Enantiomeren- überschuss [%ee]** |
|---|---|---|---|---|---|
| 0,1 | 25 | 25 | Acetophenon | 40 % | 58 |
| 0,1 | 25 | 100 | Acetophenon | 77% | 58 |

Reaktionsmedium: 2,0 g Acetophenon, 5mM Lösung von Kaliumisopropoxid in 16,6 ml Isopropanol

## Patentansprüche

1. Verbindungen der Formel (I), 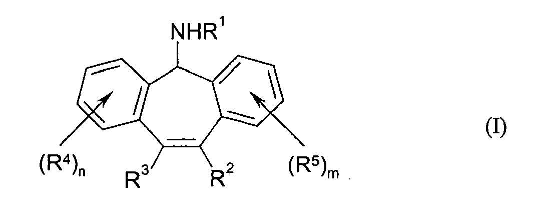 in der
R¹ entweder für einen Rest der Formel (II) steht 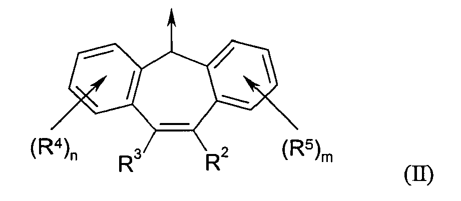
oder für einen Rest der Formel (III), 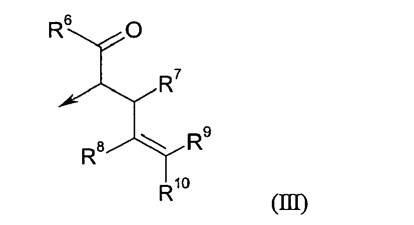 wobei in den Formeln (I), (II) und (III)
die Pfeile jeweils die Bindung des gesamten Restes zum Stickstoffatom anzeigen oder, sofern sie in die Mitte eines aromatischen Systems zeigen, eine Bindung der jeweiligen Reste zum aromatischen Gerüst in beliebiger Position,
R² und R³ jeweils unabhängig voneinander für Wasserstoff, Cyano, Fluor, Chlor, Brom, Iod, C₁-C₁₈-Alkyl, C₄-C₂₄-Aryl, C₅-C₂₅-Arylalkyl, CO₂M, wobei M für ein Alkalimetallion oder ein gegebenenfalls organisches Ammoniumion stehen kann, CONH₂, SO₂N(R¹¹)₂, wobei R¹¹ für Wasserstoff, C₁-C₁₂-Alkyl, C₄-C₁₄-Aryl oder C₅- C₁₅-Arylalkyl steht, SO₃M oder für Reste der Formel (IV) stehen,
T-Het-R¹² (IV)
in der
T fehlt oder für Carbonyl steht,
Het für Sauerstoff oder NR¹¹ steht,
R¹² für C₁-C₁₈-Alkyl, C₄-C₂₄-Aryl oder C₅-C₂₅-Arylalkyl oder N(R¹²)₂ als Ganzes für einen 5 oder 6 gliedrigen cyclischen Aminorest steht und
n und m jeweils unabhängig voneinander für 0, 1, 2 oder 3 stehen und
R⁴ und R⁵ jeweils unabhängig voneinander ausgewählt sind aus der Gruppe Fluor, Chlor, Brom, Iod, Nitro, freies oder geschütztes Formyl, C₁-C₁₂-Alkyl, C₁-C₁₂-Alkoxy, C₁-C₁₂-Halogenalkoxy, C₁-C₁₂- Halogenalkyl, C₄-C₁₄-Aryl, C₅-C₁₅-Arylalkyl oder Resten der Formel (V),
L-Q-T-W (V)
in der unabhängig voneinander
L fehlt oder für C₁-C₈-Alkylen oder C₂-C₈-Alkenylen steht und
Q fehlt oder für Sauerstoff, Schwefel oder NR¹¹ steht,
T für eine Carbonyl-Gruppe steht und
W für R¹¹, OR¹¹, NHR¹² oder N(R¹²)₂ steht, ,
wobei N(R¹²)₂ weiterhin als Ganzes für einen 5 oder 6 gliedrigen cyclischen Aminorest stehen kann
oder Resten der Formeln (VIa-g)
| | | | |
|---|---|---|---|
| L-W | (VIa) | L-SO₂-W | (VIb) |
| L-NR¹²SO₂R¹² | (VIc) | L-SO₃Z | (VId) |
| L-PO₃Z₂ | (VIe) | L-COZ | (VIf) |
| L-CN | (VIg) | | |
in denen L, Q, W und R¹² die unter der Formel (IV) angegebene Bedeutung besitzen und Z für Wasserstoff oder M steht
und in Formel (III)
R⁶ für OR¹¹ oder N(R¹¹)₂ steht, wobei N(R¹¹)₂ zusammen auch für einen 5 oder 6 gliedrigen cyclischen Aminorest stehen kann,
R⁷ und R⁸ jeweils unabhängig voneinander für Wasserstoff, C₁-C₈-Alkyl, C₄-C₁₀- Aryl, C₅-C₁₁-Arylalkyl oder C₂-C₈-Alkenyl stehen und
R⁹ und R¹⁰ jeweils unabhängig voneinander für Wasserstoff, C₁-C₈-Alkyl, C₄-C₁₄- Aryl, C₅-C₁₁-Arylalkyl oder C₂-C₈-Alkenyl stehen oder
R⁷ und R⁹ oder R⁸ und R¹⁰ jeweils zusammen für C₃-C₁₂-Alkylen oder C₃-C₁₂-Alkenylen stehen.

2. Verfahren zur Herstellung von Verbindungen der Formel (I) gemäß Anspruch 1, **dadurch gekennzeichnet, dass** Verbindungen der Formel (IV)
H₂NR¹ (IV)
in der R¹ die in Anspruch 1 genannte Bedeutung besitzt,
mit Verbindungen der Formel (V) umgesetzt werden, 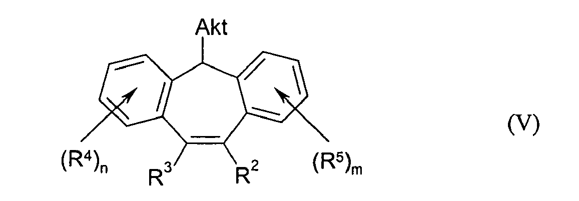 in der R², R³, R⁴, R⁵ , n und m die in Anspruch 1 genannten Bedeutungen besitzen und
Akt für Chlor, Brom, Iod, Trifluoracetyl oder einen Sulfonyloxy-Rest steht,
die dabei entstehenden Ammoniumsalze der Formel (VI) 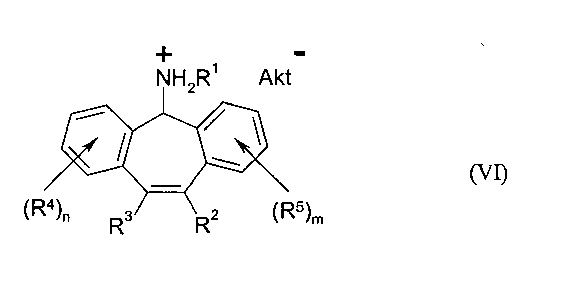 in Gegenwart von Base in Verbindungen der Formel (I) überführt werden.

3. Verbindungen der Formel (VI) gemäß Anspruch 2.

4. Übergangsmetallkomplexe von Verbindungen der Formel (I) gemäß Anspruch 1.

5. Übergangsmetallkomplexe nach Anspruch 4, **dadurch gekennzeichnet, dass** sie der Formel (VIII) gehorchen
[M⁵(I)(L)]An (VIII)
in der
M⁵ für Rhodium oder Iridium
(I) für eine Verbindung der Formel (I)
(L) für einen neutralen mono- oder bidentanten Liganden steht und
An ohne Rücksicht auf eine mögliche Koordination an das Metall für ein Anion steht.

6. Übergangsmetallkomplexe enthaltend Verbindungen der Formel (I) gemäß Anspruch 1 in der Art, dass das Stickstoffatom des zentralen Siebenringes von den Verbindungen der Formel (I) als Amid an das Metallatom koordiniert.

7. Übergangsmetallkomplexe nach Anspruch 6, **dadurch gekennzeichnet, dass** sie der Formel (X) gehorchen
[M⁶(I-)(L)] (X)
in der
M⁶ für Rhodium oder Iridium
(I-) für eine Verbindung der Formel (I) steht, in der das Stickstoffatom des zentralen Siebenringes als Amid an das Metallatom koordiniert und
(L) für einen neutralen mono- oder bidentanten Liganden steht.

8. Katalysatoren, enthaltend Übergangsmetallkomplexe gemäß Anspruch 4, 5, 6 oder 7.

9. Verwendung von Katalysatoren gemäß Anspruch 8 für Hydrogenierungen.
